(19) Europäisches Patentamt
European Patent Office
Office européen des brevets

(11) Veröffentlichungsnummer: **0 364 877 A1**

(12) # EUROPÄISCHE PATENTANMELDUNG

(21) Anmeldenummer: **89118926.8**

(51) Int. Cl.5: **C07C 315/00 , C07C 317/14**

(22) Anmeldetag: **12.10.89**

(30) Priorität: **19.10.88 DE 3835562**

(43) Veröffentlichungstag der Anmeldung:
**25.04.90 Patentblatt 90/17**

(84) Benannte Vertragsstaaten:
**CH DE FR GB IT LI**

(71) Anmelder: **BASF Aktiengesellschaft**
**Carl-Bosch-Strasse 38**
**D-6700 Ludwigshafen(DE)**

(72) Erfinder: **Stumpp, Michael, Dr.**
**An der Marlach 13**
**D-6705 Deidesheim(DE)**
Erfinder: **Neumann, Peter, Dr.**
**Poststrasse 28**
**D-6800 Mannheim 31(DE)**
Erfinder: **Eilingsfeld, Heinz, Dr.**
**Pierstrasse 9a**
**D-6710 Frankenthal(DE)**

(54) **Verfahren zur Herstellung von Bis(4-chlorphenyl) sulfon.**

(57) Verfahren zur Herstellung von Bis(4-chlorphenyl)sulfon durch Umsetzung von Chlorbenzol mit Schwefelsäure bei einer Temperatur von 200 bis 250 °C, wobei man ein Kondensationshilfsmittel umsetzt.

EP 0 364 877 A1

## Verfahren zur Herstellung von Bis(4-chlorphenyl)sulfon

Die vorliegende Erfindung betrifft ein neues Verfahren zur Herstellung von Bis(4-chlorphenyl)sulfon durch Umsetzung von Chlorbenzol mit Schwefelsäure in Gegenwart eines Kondensationshilfsmittels.

Bis(4-chlorphenyl)sulfon ist ein wichtiges Zwischenprodukt, das hauptsächlich für die Herstellung aromatischer Polysulfone und zur Synthese von Bis(4-aminophenyl)sulfon, das man sowohl für die Lepratherapie als auch zur Härtung von Epoxidharzen benötigt, verwendet wird. Voraussetzung für diesen Verwendungszweck ist eine hohe Reinheit des Bis(4-chlorphenyl)sulfons.

Man kann Bis(4-chlorphenyl)sulfon z. B. nach dem in der DE-A 1 087 592 beschriebenen Verfahren durch Umsetzung von Chlorbenzol mit einer Mischung aus Schwefeltrioxid und Dimethyl- oder Diethylpyrosulfat herstellen (s.a. US-A 3 415 887). Bei diesem Verfahren wird jedoch Dimethylsulfat verwendet, das wegen seiner Toxizität auf Bedenken stößt.

Die bekannteste Methode zur Herstellung von Bis(4-chlorphenyl)sulfon ist die Friedel-Crafts-Reaktion von 4-Chlorbenzolsulfonsäurechlorid mit Chlorbenzol, wobei man als Katalysator z. B. Eisen(III)chlorid verwendet. Bei dieser Arbeitsweise, die z. B. in der DE-A 2 704 972 oder der US-A 4 172 852 beschrieben ist, ist die Verwendung von Eisen(III)chlorid von Nachteil. Die Umsetzung zum Sulfon wird in Chlorbenzol als Lösungsmittel bei ca. 140 °C durchgeführt. Aus J. Am. Chem. Soc. 76, 5491 (1984), ist jedoch bekannt, daß Eisen(III)chlorid bei dieser Temperatur auch als Chlorierungsmittel auf Chlorbenzol wirkt, so daß als Nebenprodukte erhebliche Anteile an Dichlorbenzolen entstehen, die eine aufwendige Aufarbeitung notwendig machen, da auch Dichlorbenzole mit Chlorbenzolsulfonsäurechlorid zu unerwünschten Sulfonen reagieren können.

Wird die Reaktion, ausgehend von Chlorbenzol, als Einstufenreaktion ohne Isolierung des Zwischenprodukts Chlorbenzolsulfonsäurechlorid durchgeführt, so ist darauf zu achten, daß in der Reaktionsmischung keine Sulfonsäure, kein Thionylchlorid und keine Schwefelchloride (als Verunreinigung des Thionylchlorids) mehr vorhanden sind, da die freie Sulfonsäure zur Desaktivierung des Katalysators und Thionylchlorid sowie Schwefelchloride ebenfalls zu unerwünschten Nebenprodukten führen. Erschwerend kommt hinzu, daß man zur vollständigen Umsetzung von Chlorbenzolsulfonsäure mit Thio nylchlorid zum entsprechenden Sulfonsäurechlorid N,N-Dimethylformamid benötigt, wobei das cancerogene N,N-Dimethylcarbamoylchlorid als Nebenprodukt entsteht. Zudem muß anschließend das Eisenchlorid aus der Reaktionsmischung durch Hydrolyse entfernt werden. Dabei erhält man einerseits ein sehr korrosives Medium, das hohe Anforderungen an die verwendeten Werkstoffe stellt, andererseits gelangen auf diesem Weg auch größere Mengen an Chlorbenzol in die wäßrige Phase, wodurch eine Aufarbeitung des Abwassers erforderlich wird.

In der DE-A 2 252 571 wird die Synthese von Bis(4-chlorphenyl)sulfon aus Chlorbenzol und Chlorbenzolsulfonsäure beschrieben. Bei einer Temperatur von 220 bis 260 °C und überatmosphärischem Druck erhält man Bis(4-chlorphenyl)sulfon in guter Ausbeute allerdings bei verhältnismäßig langen Reaktionszeiten.

Aufgabe der vorliegende Erfindung war die Bereitstellung eines im Hinblick auf Selektivität und Raum-Zeit-Ausbeute verbesserten Verfahrens zur Herstellung von Bis(4-chlorphenyl)sulfon aus Chlorbenzol und Schwefelsäure.

Diese Aufgabe wurde dadurch gelöst, daß man bei der Herstellung von Bis(4-chlorphenyl)sulfon durch Erhitzen eines Gemisches aus Chlorbenzol und Schwefelsäure auf eine Temperatur von 200 bis 250 °C ein Kondensationshilfsmittel zusetzt.

Geeignete Kondensationshilfsmittel sind z.B. Borsäure oder Trifluormethansulfonsäure.

Die Bildung von Bis(4-chlorphenyl)sulfon aus Chlorbenzol und Schwefelsäure verläuft in zwei Kondensationsschritten:

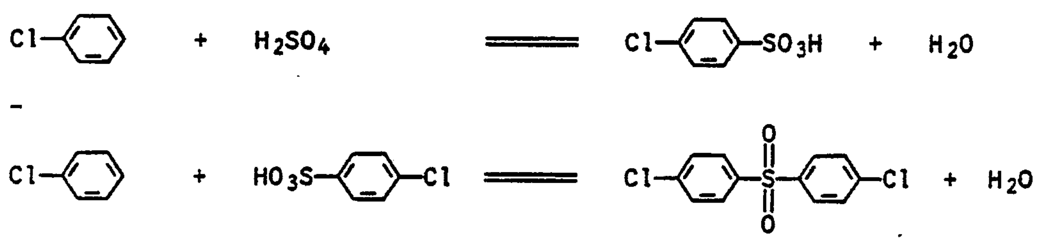

(I)

(II)

Wie allgemein bekannt und z. B. in der DE-A 2 252 571 beschrieben, verläuft der erste Kondensationsschritt, d. h. die Sulfonierungsreaktion (I), sehr viel schneller als die Sulfonbildung (II). Aufgrund der

geringen Reaktivität von Chlorbenzol erfordern jedoch beide Kondensationsschritte drastische Reaktionsbedingungen. Wie in J. Org. Chem. 32, 2931 (1967), beschrieben, wird bei einer Temperatur von 80 °C, auch bei Zusatz von Polyphosphorsäure, keine Umsetzung von Chlorbenzol mit p-Toluolsulfonsäure beobachtet.

Der Einfluß der Reaktionstemperatur auf die Bildung von Bis(4-chlorphenyl)sulfon ist auch in der DE-A 2 252 571 beschrieben. Allerdings verläuft die Reaktion bei 220 bis 260 °C so langsam, daß eine lange Reaktionszeit für einen vernünftigen Umsatz notwendig ist.

Erfindungsgemäß läßt sich nun die Reaktionszeit beträchtlich reduzieren, wenn man dem Reaktionsgemisch ein Kondensationshilfsmitteln, insbesondere in katalytischen Mengen, zusetzt.

Da die Kondensationsschritte (I) und (II) reversibel sind, ist es von Vorteil, das bei der Reaktion entstehende Wasser durch azeotrope Destillation dem Reaktionsgemisch zu entziehen. Das Schleppmittel wird im allgemeinen danach kontinuierlich in das Reaktionsgemisch zurückgeführt. Als Schleppmittel dient in der Regel Chlorbenzol.

Die Reaktionsbedingungen werden vorteilhaft so gewählt, daß ein Druck von 4 bis 5 bar eingehalten wird. Zur Aufrechterhaltung die Siedebedingungen wird die Temperatur während der azeotropen Destillation kontinuierlich von 200 bis auf 250 °C erhöht. Die Aufrechterhaltung der Siedebedingungen kann jedoch auch isotherm durch Verringerung des Drucks bei konstanter Temperatur erreicht werden.

Durch Zusatz von katalytischen Mengen an Borsäure verkürzt sich z.B. die Reaktionszeit bei 68 %igem Umsatz um 10 Stunden (6 Stunden im Vergleich zu 16 Stunden ohne Kondensationshilfsmittel), nach 10 Stunden Reaktionszeit erhält man einen Umsatz von 84 %.

Ähnlich verhält es sich bei Zusatz von Trifluormethansulfonsäure.

Überraschenderweise wurde außerdem gefunden, daß der Zusatz eines Kondensationshilfsmittels auch einen vorteilhaften Einfluß auf die Isomerenverteilung der gebildeten Diphenylsulfone ausübt. Folgende Isomerenverteilungen wurden gefunden:

| | 4,4′- | 2,4′- | 3,4′-Isomer |
|---|---|---|---|
| ohne Zusatz eines Kondensationsmittels | 79 % | 7,5 % | 13,5 % |
| mit Zusatz von Borsäure | 84,8 % | 7,8 % | 7,4 % |
| mit Zusatz von Trifluormethansulfonsäure: | 89,3 % | 5,2 % | 5,5 % |

Durch Zusatz von Borsäure oder Trifluormethansulfonsäure kann daher vor allem der Anteil des 3,4′-Isomeren reduziert werden.

Bezogen auf die eingesetzte Menge an Schwefelsäure verwendet man im allgemeinen stöchiometrische oder überschüssige Mengen an Chlorbenzol.

Die Menge des Katalysators beträgt in der Regel 0,01 bis 20 mol-% bezogen auf 1 mol Schwefelsäure. Besonders vorteilhaft verläuft die Reaktion unter Verwendung von 5 bis 10 mol-% Borsäure oder 0,05 bis 0,5 mol-% Trifluormethansulfonsäure, jeweils bezogen auf 1 mol Schwefelsäure.

Die Reaktionsprodukte können wie üblich abgetrennt werden, und das Bis(4-chlorphenyl)sulfon kann nach an sich bekannten Verfahren, beispielsweise durch selektives Waschen mit einem Lösungsmittel, durch fraktionierte Kristallisation oder durch Zentrifugation, gereinigt werden.

Weitere Einzelheiten des erfindungsgemäßen Verfahrens können den folgenden Beispielen entnommen werden.

Beispiel 1

In einem 5-1-Reaktionskessel aus Hasteloy C 4 wurden 2,03 kg Chlorbenzol und 0,917 kg Schwefelsäure (96 gew.-%ig) vorgelegt und unter Eigendruck auf 200 °C erwärmt (4,5 bar). Durch öffnen des Reduzierventils wurde das entstehende Chlorbenzol-Wasserdampf-Gemisch entspannt, kondensiert und das Chlorbenzol in die Reaktionslösung zurückgeführt. Durch kontinuierliche Erhöhung der Temperatur auf 240 °C wurde der Druck auf 4,5 bis 5 bar unter Siedebedingungen gehalten. Nach 16 Stunden Reaktionszeit wurde auf Raumtemperatur abgekühlt, das restliche Chlorbenzol durch Destillation entfernt und der Rückstand in 10 l Wasser ausgerührt, der verbleibende Feststoff abfiltriert und zwei Mal mit 1 l Wasser gewaschen und getrocknet.
Ausbeute: 1,76 kg; 68 (Sulfone gesamt)
Isomerenanteil: 4,4′: 79 %

2,4′: 7,5 %
3,4′: 13,5 %

Beispiel 2

Analog Beispiel 1, jedoch mit Zusatz von 30 g Borsäure und 10 Stunden Reaktionszeit.
Ausbeute: 2,17 kg (84 %)
Isomerenanteil: 4,4′: 84,8 %
2,4′: 7,8 %
3,4′ : 7,4 %

Beispiel 3

Analog Beispiel 1, jedoch mit Zusatz von 5 ml Trifluormethansulfonsäure und 10 Stunden Reaktionszeit.
Ausbeute: 1,94 kg (75 %)
Isomerenanteil: 4,4′: 89,3 %
2,4′: 5,2 %
3,4′: 5,5 %

## Ansprüche

1. Verfahren zur Herstellung von Bis(4-chlorphenyl)sulfon durch Umsetzung von Chlorbenzol mit Schwefelsäure bei einer Temperatur von 200 bis 250 °C, dadurch gekennzeichnet, daß man ein Kondensationshilfsmittel zusetzt.

2. Verfahren gemäß Anspruch 1, dadurch gekennzeichnet, daß man Borsäure oder Trifluormethansulfonsäure als Kondensationshilfsmittel zusetzt.

3. Verfahren gemäß Anspruch 1, dadurch gekennzeichnet, daß man 5 bis 10 mol-% Borsäure oder 0,05 bis 0,5 mol-% Trifluormethansulfonsäure, jeweils bezogen auf 1 Mol Schwefelsäure, zusetzt.

4. Verfahren gemäß Anspruch 1, dadurch gekennzeichnet, daß man das bei der Reaktion entstehende Wasser azeotrop aus dem Reaktionsgemisch entfernt.

Europäisches Patentamt

# EUROPÄISCHER RECHERCHENBERICHT

Nummer der Anmeldung

EP 89 11 8926

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X,Y | US-A-3 579 590 (D.S. DAVIS et al.) * Zusammenfassung; Spalte 3, Linien 46,47; Spalte 3, Linien 56-75; Ansprüche 1-4 * | 1-4 | C 07 C 315/00<br>C 07 C 317/14 |
| A | EP-A-0 147 298 (RHONE-POULENC) * Seite 6; Beispiel 3; Ansprüche 1,2 * | 1 | |
| A | DE-A-3 723 401 (NEW JAPAN CHEMICAL) * Spalte 4; Linien 40-50; Spalte 9, Beispiel 4; Ansprüche 1-3,7 * | 1 | |
| A | DE-A-1 950 394 (CHRIS-CRAFT INDUSTRIES) * Seite 14, Beispiel 1; Anspruch 1 * | 1 | |
| A,D | DE-A-2 252 571 (ICI) * Seite 3, Beispiel 1; Ansprüche 1-5 * | 1 | |
| Y | US-A-3 870 685 (R.S. JONES et al.) * Zusammenfassung; Anspruch 1 * | 1-4 | |

RECHERCHIERTE SACHGEBIETE (Int. Cl.5)

C 07 C 315/00
C 07 C 317/00

Der vorliegende Recherchenbericht wurde für alle Patentansprüche erstellt

| Recherchenort | Abschlußdatum der Recherche | Prüfer |
|---|---|---|
| BERLIN | 15-01-1990 | RUFET J.M.A. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)